(19) Europäisches Patentamt European Patent Office Office européen des brevets

(11) **EP 4 751 654 A1**

(12) **EUROPEAN PATENT APPLICATION**

(43) Date of publication:
**03.06.2026 Bulletin 2026/23**

(21) Application number: **25217572.4**

(22) Date of filing: **21.11.2025**

(51) International Patent Classification (IPC):
**A61B 7/04** (2006.01)

(52) Cooperative Patent Classification (CPC):
**A61B 7/04; G01H 9/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **02.12.2024 JP 2024209752**

(71) Applicant: **Canon Kabushiki Kaisha
Tokyo, 146-8501 (JP)**

(72) Inventor: **SASAOKA, Osamu
Tokyo, 146-8501 (JP)**

(74) Representative: **TBK
Bavariaring 4-6
80336 München (DE)**

(54) **DETECTION APPARATUS AND ELECTRONIC STETHOSCOPE**

(57) A detection apparatus is configured to detect vibration of a subject. The detection apparatus includes a diaphragm (206) configured to be displaced in response to vibration of the subject, a light emitter (202) configured to emit light toward the diaphragm, a photodetector (204) configured to receive light reflected by the diaphragm and generate a signal corresponding to the reflected light, a casing (208) configured to internally house the light emitter and the photodetector, and a grip (120) configured to rotate about a rotation shaft (1001b) relative to the casing, the grip including a portion grippable by a user. The light emitter and the photodetector are respectively arranged at one end and another end of the casing with respect to an axial direction of the rotation shaft.

**F I G. 14**

EP 4 751 654 A1

**Description**

TECHNICAL FIELD

The present disclosure relates to a detection apparatus and an electronic stethoscope.

BACKGROUND

Electronic stethoscopes for obtaining vibration sound based on the displacement of a body surface are becoming more common. Japanese Patent Laid-Open No. 2022-119446 proposes an electronic stethoscope that gathers body sounds via a capacitive microphone. Japanese Patent Laid-Open No. 2017-47095 proposes a stethoscope that uses a vibration sensor in an obtaining unit that obtains body sounds.

SUMMARY

A detection apparatus such as an electronic stethoscope is preferably compact so that it can be used in various places and is convenient to carry. An aspect of the present disclosure provides technology for reducing the size of a detection apparatus.

The present disclosure in its first aspect provides a detection apparatus as specified in claim 1. Optional features are specified in claim 2 to 9.

The present disclosure in its second aspect provides an electronic stethoscope as specified in claim 10.

The present disclosure in its third aspect provides a detection apparatus as specified in claim 11. Optional features are specified in claim 12 to 14.

The present disclosure in its fourth aspect provides an electronic stethoscope as specified in claim 15.

Features of the present disclosure will become apparent from the following description of embodiments with reference to the attached drawings. The following description of embodiments is described by way of example.

BRIEF DESCRIPTION OF THE DRAWINGS

The accompanying drawings, which are incorporated in and constitute a part of the specification, illustrate embodiments of the present disclosure, and together with the description, serve to explain the principles of the embodiments.

FIGS. 1A and 1B are perspective views for describing the external appearance of an electronic stethoscope according to some embodiments.
FIG. 2 is a schematic view for describing the configuration of a chest piece according to some embodiments.
FIGS. 3A and 3B are schematic views for describing the operation of the chest piece according to some embodiments.
FIG. 4 is a schematic view for describing movement of reflected light according to some embodiments.
FIG. 5 is a diagram for describing the relationship between displacement amount and displacement signal according to some embodiments.
FIG. 6 is a block diagram for describing the configuration of an electronic stethoscope according to some embodiments.
FIGS. 7A and 7B are schematic views for describing the operation of the chest piece according to some embodiments.
FIG. 8 is a schematic view for describing change of a light-receiving range according to some embodiments.
FIGS. 9A and 9B are schematic views for describing deformation of the chest piece according to some embodiments.
FIGS. 10A and 10B are perspective views for describing the external appearance of the chest piece according to some embodiments.
FIGS. 11A and 11B are perspective views for describing the external appearance of a grip according to some embodiments.
FIGS. 12A and 12B are perspective views for describing the external appearance of the electronic stethoscope according to some embodiments.
FIGS. 13A to 13C are diagrams for describing the shape of the electronic stethoscope according to some embodiments.
FIG. 14 is a cross-sectional view for describing the shape of the electronic stethoscope according to some embodiments.
FIGS. 15A and 15B are cross-sectional views for describing a pressing surface of the electronic stethoscope according to some embodiments.
FIG. 16 is a cross-sectional view for describing a modification example of the shape of the electronic stethoscope according to some embodiments.

## DESCRIPTION OF THE EMBODIMENTS

Hereinafter, embodiments will be described in detail with reference to the attached drawings. Note, the following embodiments are not intended to limit the scope of the claims. Multiple features are described in the embodiments, but it is not the case that all such features are required, and multiple such features may be combined as appropriate. Furthermore, in the attached drawings, the same reference numerals are given to the same or similar configurations, and redundant description thereof is omitted.

External Appearance of Electronic Stethoscope

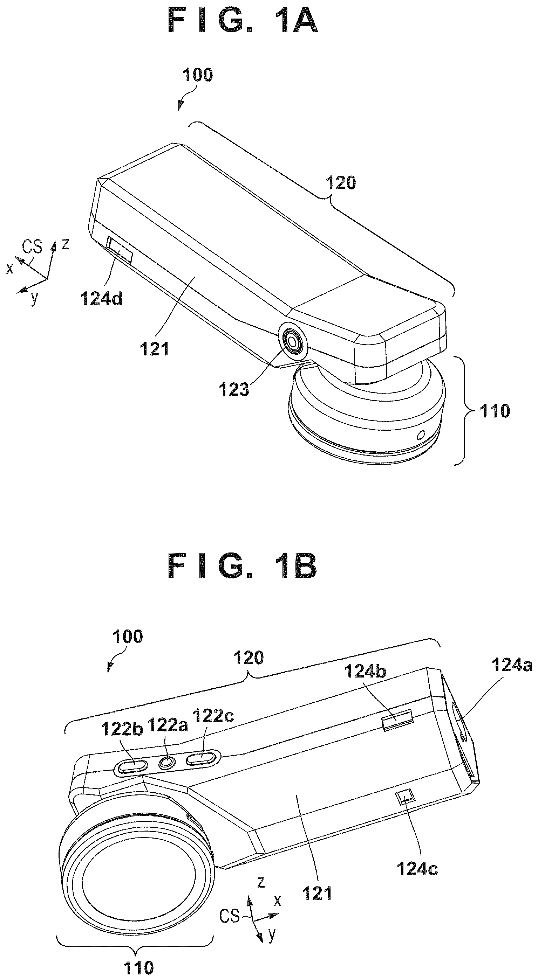

The external appearance of an electronic stethoscope 100 according to some embodiments will now be described with reference to FIGS. 1A and 1B. For describing directions, a coordinate system CS has been provided in each of the following diagrams. The coordinate system CS is a three-dimensional Cartesian coordinate system with an x-axis, a y-axis, and a z-axis orthogonal to one another. Hereinafter, the z-axis positive direction may be expressed as up/upper and the z-axis negative direction may be expressed as down/lower. FIG. 1A illustrates the external appearance of the electronic stethoscope 100 as seen from a certain direction. FIG. 1B illustrates the external appearance of the electronic stethoscope 100 as seen from a different direction. The electronic stethoscope 100 is an example of a detection apparatus that detects vibration in a subject. The electronic stethoscope 100 may be used in auscultation of a target body, for example, and in this case, the subject is a target body. A target body may be a human or an animal other than a human. In the example described below, the subject is a target body, but the subject may be an object other than a target body.

The electronic stethoscope 100 includes a chest piece 110 and a grip 120. The chest piece 110 is a portion that comes into contact with the target body when the electronic stethoscope 100 is used for a diagnosis. The chest piece 110 generates a displacement signal indicating a displacement amount of a body surface. The chest piece 110 is used for detecting the displacement of a body surface and thus may be referred to as a displacement detection apparatus. Also, the chest piece 110 is used for detecting vibration of the body surface and thus may be referred to as a body-surface vibration detection apparatus.

The grip 120 includes a portion gripped by the user when the user (for example, a physician, a nurse, or a public health nurse) using the electronic stethoscope 100 presses the chest piece 110 against the body surface. Hereinafter, the user of the electronic stethoscope 100 may be simply referred to as the user. The grip 120 may be referred to as a handle, a handgrip, a grip, or the like. The chest piece 110 is joined to the grip 120. Specifically, the grip 120 has a rod-like shape, and the chest piece 110 is joined to one end of the grip 120. The chest piece 110 is pivotable with respect to the grip 120. Alternatively, the chest piece 110 may be fixed to the grip 120.

The grip 120 includes a casing 121. The grip 120 includes a battery (not illustrated) and a circuit board (not illustrated) inside the casing 121. The battery stores the operation power of the electronic stethoscope 100. The circuit board includes a circuit element for controlling the operation of the electronic stethoscope 100. The grip 120 includes operation portions 122a, 122b, 122c; a power switch 123; and connectors 124a, 124b, 124c, and 124d on the outer surface of the casing 121.

The operation portions 122a, 122b, 122c receive operations from the user. In the example of FIG. 1B, the operation portions 122a, 122b, 122c are each buttons. At least some of the buttons (122a and 122b) are provided at a position overlapping a diaphragm 206 in the long side direction of the grip 120. The operation portion 122a is a button for switching the operation mode of the electronic stethoscope 100. The electronic stethoscope 100 can switch between a plurality of operation modes including a cardiac sound mode, a respiratory sound mode, and a low-power mode and operate. The cardiac sound mode is an operation mode for listening to cardiac sounds. The respiratory sound mode is an operation mode for listening to respiratory sounds. The low-power mode is an operation mode that consumes less power than the cardiac sound mode and the respiratory sound mode. The operation portions 122b and 122c are buttons for adjusting the gain of the sound signal output by the electronic stethoscope 100. By adjusting the gain of the sound signal, the volume of the sound output from the electronic stethoscope 100 (via an earphone, for example) is adjusted. The operation portions 122a, 122b, 122c may be physical buttons or software buttons displayed on a touch panel.

The operation portions 122a, 122b, 122c are disposed on the outer surface of the casing 121 at or near the chest piece 110. Due to this arrangement, the user can operate the operation portions 122a, 122b, and 122c (for example, using the thumb if the grip 120 is gripped by the right hand and using the index finger if the grip 120 is gripped by the left hand) in a state where the user is gripping the grip 120 while using the electronic stethoscope 100.

The grip 120 may include a plurality of indicators that display the state of the electronic stethoscope 100 by lighting up. At least some of the operation portions 122a, 122b, 122c may function as such an indicator. The grip 120 may include one or more light-emitting diodes (LEDs). The plurality of indicators may include an indicator that indicates whether the power of the electronic stethoscope 100 is on or off. The plurality of indicators may include an indicator that indicates the current operation mode of the electronic stethoscope 100. The plurality of indicators may include an indicator that indicates whether or not the electronic stethoscope 100 is wirelessly connected to an external apparatus. The plurality of indicators may include an indicator that indicates whether or not the chest piece 110 is pressed against a body surface.

The power switch 123 is a switch for switching the power of the electronic stethoscope 100 on and off. The connectors 124a, 124b, 124c, and 124d are connectors for receiving a cable or a connector of an external apparatus. Via the connectors 124a, 124b, 124c, and 124d, power may be supplied from an external apparatus to the electronic stethoscope 100 (specifically, to the battery). Also, the connectors 124a, 124b, 124c, and 124d may be used for the electronic stethoscope 100 to output a sound signal to an external apparatus.

The power switch 123 may be provided on the chest piece 110 instead of on the grip 120. At least one of the connectors 124a, 124b, 124c, and 124d may be provided on the chest piece 110 instead of on the grip 120. Also, the electronic stethoscope 100 may not include at least one of the connectors 124a, 124b, 124c, and 124d. In this case, the electronic stethoscope 100 may include a wireless charging function and may be configured with a replaceable battery.

In the example of FIGS. 1A and 1B, the electronic stethoscope 100 includes both the chest piece 110 and the grip 120. Alternatively however, the electronic stethoscope 100 may include the chest piece 110 but not include the grip 120. In this case, the chest piece 110 may be supplied with power via a cable (not illustrated), and a sound signal may be output to an external apparatus (for example, an earphone) via this cable or a different cable. Alternatively however, the chest piece 110 may include a built-in battery and wireless communication module and may output a sound signal to an external apparatus (for example, an earphone or a computer) via the wireless communication module.

In the example of FIGS. 1A and 1B, the electronic stethoscope 100 includes both the chest piece 110 and the grip 120, and the chest piece 110 is connected to the grip 120. Alternatively however, the chest piece 110 may be incorporated into the grip 120. In this case, the portion such as the diaphragm where the chest piece 110 is attached is attached to the grip 120.

Cross-sectional Configuration of Chest Piece of Electronic Stethoscope

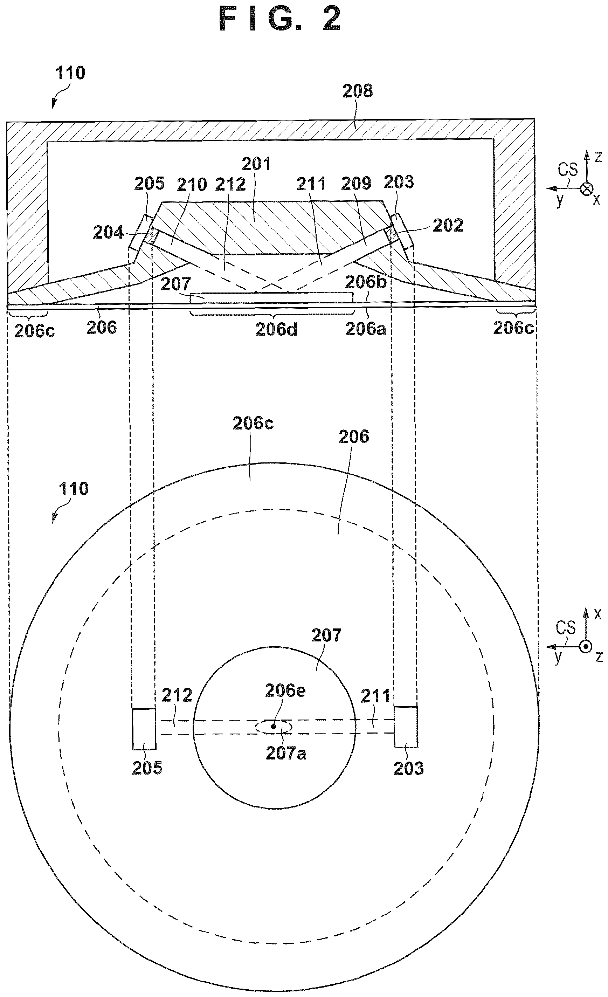

An example of the configuration of the chest piece 110 will now be described with reference to FIG. 2. The upper side of FIG. 2 illustrates a cross-sectional view of the chest piece 110, and the lower side of FIG. 2 illustrates a plan view of the chest piece 110. In the plan view, to make clear the positional relationship between the component elements, only a light emitter circuit board 203, a photodetector circuit board 205, the diaphragm 206, and a reflective medium 207 are illustrated. The chest piece 110 may include, in addition to the component elements illustrated in FIG. 2, a circuit board mounted with a circuit element for controlling the operation of the chest piece 110.

The chest piece 110 includes a holding member 201, a light emitter 202, the light emitter circuit board 203, a photodetector 204, the photodetector circuit board 205, the diaphragm 206, the reflective medium 207, and a casing 208. The chest piece 110 may also include other component elements than these. The casing 208 internally houses the holding member 201, the light emitter 202, the light emitter circuit board 203, the photodetector 204, the photodetector circuit board 205, and the reflective medium 207. Apertures 209 and 210 are also formed in the holding member 201, and thus the casing 208 also internally houses the apertures 209 and 210. The diaphragm 206 forms a part of the outer sheath of the electronic stethoscope 100 with the casing 208.

The light emitter 202 is a light source that emits light and is a light-emitting diode (LED). The power supplied to the light emitter 202 is supplied from a power supply (the battery of the grip 120) external to the chest piece 110. The light emitter 202 is mounted on the light emitter circuit board 203. A peripheral circuit for regulating the amount of light emitted by the light emitter 202 and a power supply terminal for receiving supply of power from the external power supply of the chest piece 110 are mounted on the light emitter circuit board 203. The light emitter circuit board 203 may be a printed circuit board such as a flexible circuit board, may be a paper phenol substrate, may be a glass epoxy substrate, or may be a different substrate. The light emitter circuit board 203 including the light emitter 202 functions as a light-emitting unit.

The photodetector 204 uses the power supplied from the battery housed inside the grip 120 to generate an electrical signal based on the received light level. The power supplied to the photodetector 204 is supplied from the battery of the grip 120. The photodetector 204 is a phototransistor, a complementary metal oxide semiconductor (CMOS) sensor, or the like, for example. The photodetector 204 is mounted on the photodetector circuit board 205. In addition to the photodetector 204, a peripheral circuit for reading out signals from the photodetector 204, a signal terminal for outputting signals to an apparatus external to the chest piece 110, and a power supply terminal for receiving supply of power from the external power supply of the chest piece 110 are mounted on the photodetector circuit board 205. The photodetector circuit board 205 may be a printed circuit board such as a flexible circuit board, may be a paper phenol substrate, may be a glass epoxy substrate, or may be a different substrate. The photodetector circuit board 205 including the photodetector 204 functions as a light-receiving unit.

The holding member 201 holds the light emitter circuit board 203 and the photodetector circuit board 205. For example, each of the light emitter circuit board 203 and the photodetector circuit board 205 is fixed to the holding member 201. Fixing these circuit boards to the holding member 201 may be performed using an adhesive or using a fastening member such as a screw.

The diaphragm 206 is held by the holding member 201 and is arranged to be able to be brought into contact with a body surface. The diaphragm 206 includes an outer surface 206a that comes into contact with a body surface and an inner surface 206b, which is the surface opposite the contact surface 206a. Also, the diaphragm 206 includes a fixing portion 206c that is fixed to the holding member 201. The fixing portion 206c is located on the outer peripheral portion of the diaphragm 206. The part of the diaphragm 206 on the inner side of the fixing portion 206c is not fixed to the holding member 201. Thus, the diaphragm 206 elastically deforms when it is pressed by a subject in contact with the contact surface 206a. Specifically, the diaphragm 206 vibrates in the z-axis direction with the fixing portion 206c acting as a node. The reflective medium 207 described below is provided on the inner surface 206b of the diaphragm 206. The diaphragm 206 is a layered plate of glass epoxy resin laminate formed by impregnating glass fiber with epoxy resin and then performing a thermal curing process.

The reflective medium 207 reflects light emitted from the light emitter 202. The reflective medium 207 is bonded to the inner surface 206b of the diaphragm 206 and its movement is linked to the vibration of the diaphragm 206 pressed against a body surface so that it moves in the z-axis direction integrally with the diaphragm 206. The reflective medium 207 has a circular outer edge in a plan view. The reflective medium 207 has a diameter ranging from 15 mm to 20 mm and is arranged at a position covering a region 206d including a center 206e of the circle of the diaphragm 206. Since the displacement of the diaphragm 206 is the greatest at the center 206e, the displacement of the diaphragm 206 can be detected with high sensitivity there due to the light from the light emitter 202 reflecting at the region including the center 206e. The reflective medium 207 is made of an aluminum vapor deposition film, for example.

The light emitter 202 emits light at the inner surface 206b of the diaphragm 206. The upper surface of the reflective medium 207 reflects light emitted from the light emitter 202. In other words, the upper surface of the reflective medium 207 functions as a reflective surface. Hereinafter, light reflecting at the upper surface (reflective surface) of the reflective medium 207 may be simply referred to as light reflecting at the reflective medium 207. The reflective medium 207 performs specular reflection (mirror-like reflection) of the light emitted from the light emitter 202. Hereinafter, the light from the light emitter 202 to the reflective medium 207 may be expressed as incident light 211, and the incident light 211 after being reflected may be expressed as reflected light 212.

In the example of FIG. 2, the reflective medium 207 is a separate member to the diaphragm 206. The reflective medium 207 may be a reflective material applied to the diaphragm 206 or may be a reflective material adhered to the diaphragm 206. Alternatively, at least a portion of the inner surface 206b of the diaphragm 206 may function as a reflecting portion. For example, the entire inner surface 206b of the diaphragm 206 may have a high reflectance so that enough light can be reflected to be detectable by the photodetector 204. Alternatively, a region of the inner surface 206b of the diaphragm 206 where light emitted by the light emitter 202 reaches may have this high reflectance.

In a state where the diaphragm 206 is not in contact with a body surface, the light emitter 202 is arranged so that light is emitted toward a region 207a of the reflective medium 207 including a portion that covers the center 206e of the diaphragm 206. In a state where the diaphragm 206 is not in contact with a body surface, the diaphragm 206 is flat. The light emitter 202 emits light toward a specific region (for example, the region 207a) of the reflective medium 207. As described above, an LED that emits diffused light is used as the light emitter 202. Thus, the chest piece 110 includes the aperture 209 to regulate the light emitted from the light emitter 202. Via the aperture 209, only a portion of the light emitted from the light emitter 202 is incident on the reflective medium 207. In the example of FIG. 2, the portion of the holding member 201 formed with an opening corresponds to the aperture 209.

The photodetector 204 is arranged to receive the reflected light 212. Specifically, the photodetector 204 is arranged at a position at which the received light amount of the reflected light 212 changes due to vibration of the diaphragm 206 in the z-axis direction. The photodetector 204 is arranged so that more light is received at the reflected light 212 in a state where the diaphragm 206 is not in contact with a body surface (in other words, a state where the diaphragm 206 is flat) than when the diaphragm 206 is vibrating. In other words, the photodetector 204 may output an electrical signal corresponding to the amount of the reflected light 212 received and may determine the displacement amount of the diaphragm 206 on the basis of the electrical signal. This principle will be described below. The chest piece 110 includes the aperture 210 that regulates the light specularly reflected by the reflective medium 207. The aperture 210 reduces the amount of diffuse reflected light being incident on the photodetector 204 and allows only at least a portion of the light from the reflective medium 207 to reach the photodetector 204. In the example of FIG. 2, the portion of the holding member 201 formed with an opening functions as the aperture 210.

The casing 208 is attached to the peripheral upper surface of the holding member 201. The casing 208 covers the light emitter circuit board 203 and the photodetector circuit board 205 and reduces the amount of ambient noise entering into the casing 208. The casing 208 is made of stainless steel, for example.

Example of Operation of Electronic Stethoscope

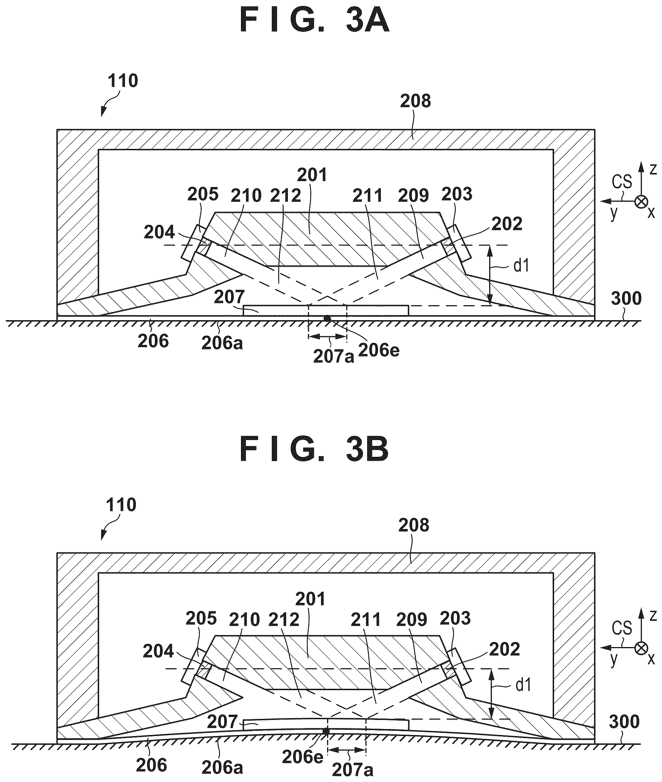

An example of the operation of the chest piece 110 of the electronic stethoscope 100 will now be described with reference to FIGS. 3A and 3B. As illustrated in FIGS. 3A and 3B, the chest piece 110 is used in a state of being in contact with a body surface 300, which is a subject. Thus, the body surface 300, the diaphragm 206, and the reflective medium 207 all vibrate together. The chest piece 110 detects the displacement in the z-axis direction of the upper surface of the reflective medium 207 as displacement in the z-axis direction of the body surface 300. The displacement of the body surface 300 occurs in response to movement of the body such as the heart beat or respiration of a human with the body surface 300. The body surface 300 is an example of a subject.

FIG. 3A illustrates a cross-sectional view of the chest piece 110 in a case where the diaphragm 206 is flat. As described above, the light emitter 202 and the photodetector 204 are arranged so that, in a state where the diaphragm 206 is flat, a larger amount of the reflected light 212 is received by the photodetector 204 than when the diaphragm 206 is vibrating. The photodetector 204 amplifies and outputs the photocurrent in accordance with the amount of received light. The peripheral circuit of the photodetector circuit board 205 generates an output value obtained by converting the photocurrent output from the photodetector 204 into voltage as a displacement signal and outputs the displacement signal to an external apparatus (for example, the grip 120). The displacement signal is an output value of the photodetector 204 reflecting the states and deformation at different times of the diaphragm 206.

FIG. 3B is a cross-sectional view of the chest piece 110 in a case where the body surface 300 is displaced upward. The distance between light emitter 202 and the upper surface of the reflective medium 207 is represented by d1. When the body surface 300 is displaced upward, the distance d1 decreases. In conjunction with this, the region 207a of the reflective medium 207 that the incident light 211 reaches moves toward the light emitter 202, and the reflected light 212 also moves toward the light emitter 202. Accordingly, the amount of the reflected light 212 reaching the photodetector 204 is decreased, and the value of the displacement signal generated by the photodetector circuit board 205 is decreased. In the state of FIG. 3B, since none of the reflected light 212 reaches the photodetector 204, the value of the displacement signal is ideally zero.

In this manner, in the chest piece 110, the light emitter 202 and the photodetector 204 are arranged so that the amount of light reaching the photodetector 204 changes according to the movement of the body surface 300, the diaphragm 206, and the reflective medium 207. Since the reflective medium 207 is displaced in conjunction with the displacement of the body surface 300, the displacement signal generated by the photodetector circuit board 205 represents displacement of the body surface 300.

Relationship of Displacement Amount of Diaphragm in Electronic Stethoscope and Displacement Amount at Light-receiving Position of Reflected Light

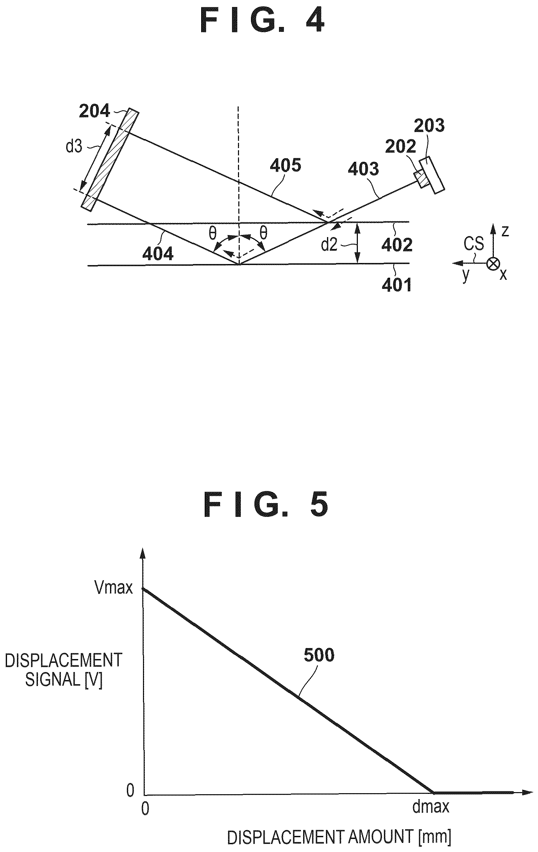

The relationship between the displacement amount of the body surface 300, the incident angle of the incident light 211, the incident angle of the reflected light 212, and the displacement amount of the position where the photodetector 204 receives the reflected light 212 with reference to FIG. 4. In FIG. 4, a position 401 indicates a reference position of the upper surface of the reflective medium 207. The upper surface of the reflective medium 207 in a case where the diaphragm 206 is flat corresponds to the reference position. A position 402 indicates a position that the upper surface of the reflective medium 207 is displaced upward from the position 401 by a displacement amount d2. Since the displacement amount d2 of the reflective medium 207 is a small amount, even if the upper surface of the reflective medium 207 is at the position 402, the upper surface of the reflective medium 207 is considered to be flat.

In FIG. 4, an optical axis 403 indicates the optical axis of the incident light 211. An incident angle of the light incident on the reflective medium 207 after being emitted from the light emitter 202 is represented by $\theta$. The incident angle $\theta$ of the incident light 211 is defined by an angle formed by the optical axis 403 of the incident light 211 and the surface normal of the upper surface of the reflective medium 207. The optical axis of the reflected light 212 when the upper surface of the reflective medium 207 is at the position 401 corresponds to an optical axis 404. The optical axis of the reflected light 212 when the upper surface of the reflective medium 207 is at the position 402 corresponds to an optical axis 405. Since the incident light 211 is specularly reflected at the upper surface of the reflective medium 207, the reflection angle of the reflected light 212 is also $\theta$. The optical axis 404 and the optical axis 405 are parallel with one another. The incident angle of the reflected light 212 with respect to the photodetector 204 is defined as $\varphi$, and $\varphi$ is 0°. The displacement amount of the position where the photodetector 204 receives the reflected light 212 in a case where the upper surface of the reflective medium 207 is displaced from the position 401 to the position 402 is represented by d3. The displacement amount d3 is defined by the displacement amount from the position where the photodetector 204 receives the optical axis 404 to the position where the photodetector 204 receives the optical axis 405. Hereinafter, the ratio of the displacement amount d3 to the displacement amount d2 is represented by a displacement gain G. In this case, the following relationship is satisfied.

$$G = 2 \times \sin\theta/\cos\varphi \ldots \quad \text{(Formula 1)}$$

Thus, even if the displacement amount d2 of the reflective medium 207 is the same, the displacement gain G also increases as the incident angle $\theta$ increases, and the displacement gain G also increases as the incident angle $\varphi$ increases.

In the example illustrated in FIG. 4, the incident angle φ is 0°. In other words, the optical axes 404 and 405 are orthogonal to the light-receiving surface of the photodetector 204. In this case, the Formula 1 becomes the following.

$$G = 2 \times \sin\theta \qquad ... \quad \text{(Formula 2)}$$

In other words, the displacement amount d3 increases as the incident angle θ increases.

In the electronic stethoscope 100, as described above, an LED is used as the light emitter 202, and the displacement of the diaphragm 206 is measured on the basis of the received light level by the photodetector 204. Alternatively, a laser light may be used as the light emitter 202, and the displacement of the diaphragm 206 may be measured on the basis of the position of the light received by the photodetector 204.

Relationship Between Displacement Amount of Body surface of Electronic Stethoscope and Displacement Signal

The relationship between the displacement amount of the body surface 300 and the displacement signal will now be described with reference to FIG. 5. The displacement signal represents the voltage output from the photodetector circuit board 205. A graph 500 of FIG. 5 represents the relationship between the displacement amount of the body surface 300 and the displacement signal. The horizontal axis of the graph 500 represents the displacement amount of the body surface 300, and the vertical axis represents the displacement signal generated by the photodetector circuit board 205.

As described above, the displacement amount of the body surface 300 is equal to the displacement amount d2 of the upper surface of the reflective medium 207. As illustrated in FIGS. 3A and 3B, as the displacement amount d3 of the reflected light 212 increases, the amount of the reflected light 212 reaching the photodetector 204 monotonically and linearly decreases. Thus, the value of the displacement signal represented by Sd is as follows.

$$Sd = Vmax - k \times d3 \qquad ... \quad \text{(Formula 3)}$$

Here, Vmax is the value of the displacement signal when the displacement amount d3 of the reflected light 212 is zero, and k is a proportional coefficient determined by the amplification factor of the amplifier circuit of the photodetector circuit board 205. By substituting d3 = G × d2 and Formula 1 into Formula 3, the following is obtained.

$$Sd = Vmax - 2k \times d2 \times \sin\theta/\cos\varphi \qquad ... \quad \text{(Formula 4)}$$

Thus, the displacement signal Sd monotonically and linearly decreases as the displacement amount d2 of the body surface 300 increases as illustrated in the graph 500. The displacement amount when the displacement signal Sd is zero is represented by dmax. When the displacement amount exceeds dmax, the reflected light 212 stops reaching the photodetector 204. Thus, even if the displacement amount d2 increases, the displacement signal Sd stays at zero. Here, the proportional coefficient k, the incident angle θ, and the incident angle φ are set so that the displacement amount d2 is in a range from 0 to dmax in an expected range for the vibration of the diaphragm 206. As illustrated in the graph 500, the light emitter 202 and the photodetector 204 are arranged so that the amount of light reaching the photodetector 204 monotonically changes in response to the reflective medium 207 moving in one direction within the operational range of the diaphragm 206.

Hardware Configuration of Electronic Stethoscope

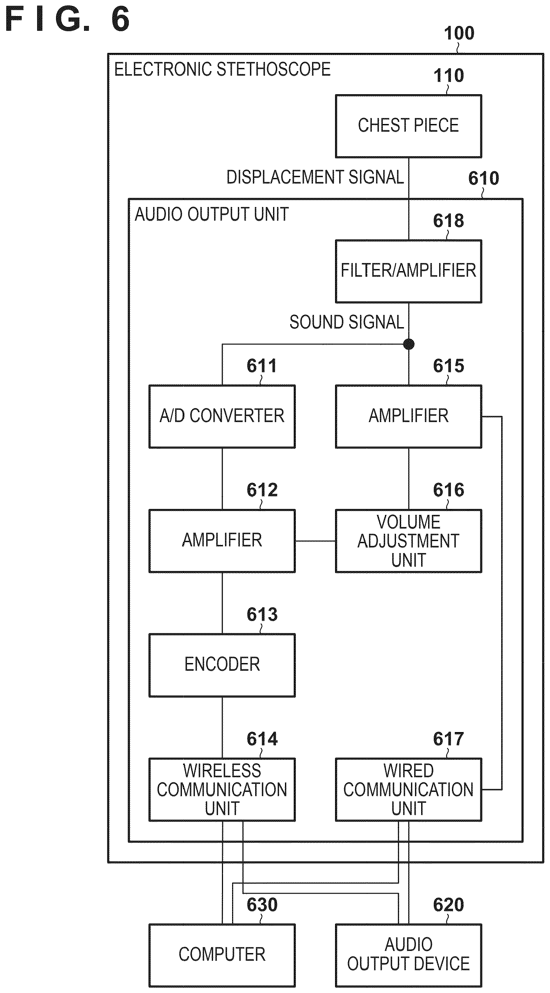

An example of the hardware configuration of the electronic stethoscope 100 will now be described with reference to FIG. 6. The electronic stethoscope 100 includes the chest piece 110 described above and an audio output unit 610. The audio output unit 610 is implemented via a plurality of circuit elements mounted on a circuit board included in the grip 120. The plurality of circuit elements include a processor. The processor constituting the audio output unit 610 transmits a sound signal based on the displacement signal generated by the chest piece 110 to an external audio output device. The sound signal transmitted by the audio output unit 610 may be referred to as a biosignal since it represents a body sound of a target body (for example, a human) with the body surface 300. The sound signal is transmitted to an audio output device 620 such as an earphone, a headphone, or the like. Also, the sound signal is transmitted to a computer 630 (for example, a personal computer, a smartphone, a tablet, or the like) at the same time as it is transmitted to the audio output device 620. A physician, nurse, or public health nurse, that is, the user, can use the audio output device 620 or the computer 630 to listen to the body sound represented by the digitally converted sound signal. The audio output device 620 is an earphone or headphone of a wired connection or wireless communication type.

The audio output unit 610 includes the component elements illustrated in FIG. 6. The audio output unit 610 can transmit sound signals via both wireless communication and wired communication due to supporting earphones and headphones as described above. In the processing described hereinafter, the audio output device 620 outputs sound signals via wired communication. The displacement signal output from the chest piece 110 is filtered and amplified at a filter/amplifier 618 and supplied to both an A/D converter 611 and an amplifier 615. The amplifier 615 further amplifies the output from the filter/amplifier 618 and supplies this to a wired communication unit 617. The wired communication unit 617 provides the amplified sound signal to the audio output device 620. The wired communication unit 617 is a 3.5 mm AUX terminal, for example. The amplification gain of the amplifier 615 is adjusted by a volume adjustment unit 616. The audio output device 620 may be configured to form a portion of the electronic stethoscope 100. In this case, the electronic stethoscope 100 includes the chest piece 110, the grip 120, and the audio output device 620.

In the processing described next, the audio output device 620 outputs sound signals via wireless communication. The A/D converter 611 converts the output from the filter/amplifier 618 to a digital signal. Thereafter, the displacement signal in a digital format is amplified at an amplifier 612 and then supplied to an encoder 613. The encoder 613 generates audio data for wireless communication by executing signal processing such as data compression, encoding, and the like on the amplified sound signal. The order of the processing of the amplifier 612 and the encoder 613 may be reversed. Thereafter, a wireless communication unit 614 compliant with a wireless communication standard such as Bluetooth (registered trademark) provides the processed audio data to the audio output device 620. The amplification gain of the amplifier 612 is adjusted by the volume adjustment unit 616. The electronic stethoscope 100 according to the example described above can output a sound signal via both wireless communication and wired communication. However, the sound signal may be able to be output via only one of these communication methods.

The transmission of a sound signal to the computer 630 is also similar to the transmission of a sound signal to the audio output device 620. The computer 630 can visually display waveform data generated on the basis of the sound signal. The waveform data may be generated by the computer 630 or may be generated by the electronic stethoscope 100. Also, a portion or all of the signal processing and the audio output processing executed by the electronic stethoscope 100 may be executed by an external apparatus (for example, the audio output device 620 or the computer 630).

The electronic stethoscope 100 can detect displacement of the body surface 300 accurately regardless of the frequency of the vibration of the body surface 300. Thus, in the case of either a relatively low frequency body sound such as a cardiac sound issued by a body from a heart beat or a relatively high frequency body sound issued by a body via respiration, good auscultation can be performed with the electronic stethoscope 100. A respiratory sound is a body-surface vibration with a frequency band including a frequency band component in a range from 500 Hz to 1 kHz, for example. A cardiac sound is a body-surface vibration with a frequency band including a frequency band component in a range from 30 Hz to 300 Hz, for example.

Detailed Example of Operation of Electronic Stethoscope

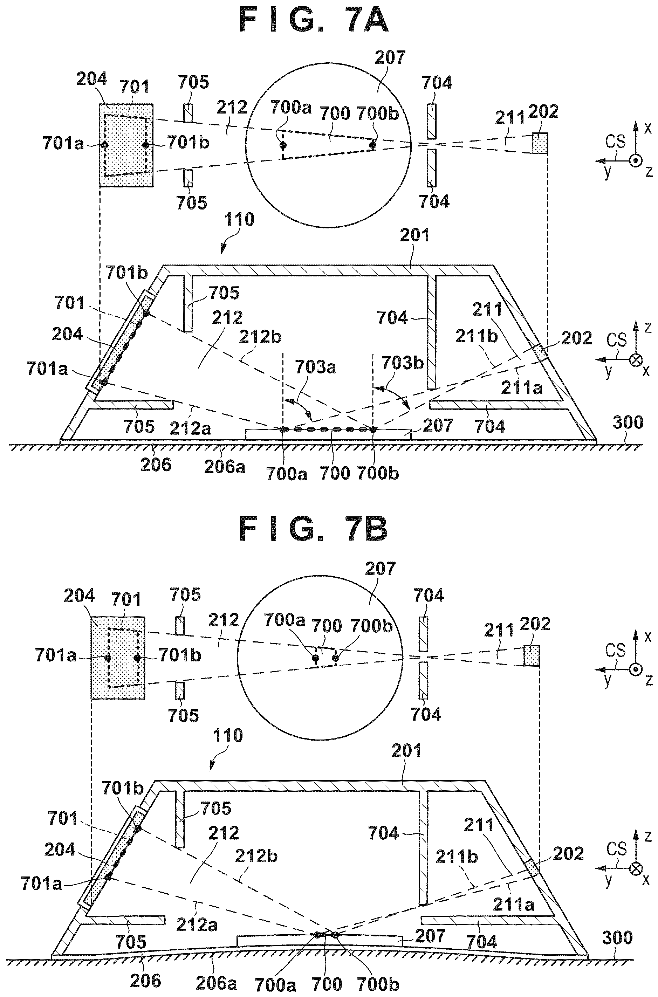

The operation of the chest piece 110 of the electronic stethoscope 100 will now be described in further detail with reference to FIGS. 7A and 7B. FIG. 7A illustrates a state (a flat state) in which the diaphragm 206 is not pressed. FIG. 7B illustrates a state in which the diaphragm 206 is pressed by the body surface 300. In both FIG. 7A and FIG. 7B, the lower side illustrates a cross-sectional view of the chest piece 110 and the upper side illustrates a plan view of the chest piece 110. In the plan view, to make clear the positional relationship between the component elements, only the light emitter 202, the photodetector 204, the reflective medium 207, a light shield 704, and a light shield 705 are illustrated.

In the chest piece 110 of FIGS. 7A and 7B, a diaphragm on the incident light 211 side is formed by the light shield 704, and a diaphragm on the reflected light 212 side is formed by the light shield 705. Thus, a portion of the light emitted by the light emitter 202 is blocked by the light shield 704 and does not reach the reflective medium 207. Also, at least a portion of the light reflected by the reflective medium 207 is dependent on the position of the reflective medium 207 and is blocked by the light shield 705.

In FIGS. 7A and 7B, the incident light 211 and the reflected light 212 are represented by a light beam reaching the photodetector 204. In FIG. 7B, a portion of the light that is emitted from the light emitter 202, passes through the opening of the light shield 704, and is reflected at the reflective medium 207 is blocked by the portion of the light shield 705 further up than the reflected light 212 and does not reach the photodetector 204.

As illustrated in FIG. 7A, the portion of the reflective medium 207 that the incident light 211 reaches when the diaphragm 206 is in a non-pressed state is referred to as an effective range 700. The effective range 700 is the portion of the reflective medium 207 that reflects the light that reaches the photodetector 204. When the diaphragm 206 is in a non-pressed state, the effective range 700 is equal to the range in which the light from the light emitter 202 reaches.

The position of the effective range 700 farthest from the light emitter 202 in the x-axis direction is represented by a far-end position 700a. In a case where a plurality of such positions exist, one of these positions is set as the far-end position 700a. The portion of the incident light 211 that reaches the far-end position 700a is represented by far incident light 211a. The far incident light 211a means that the optical path from the light emitter 202 to the reflective medium 207 is the longest. The incident angle of the incident light 211 to the reflective medium 207 corresponds to a maximum value 703a at the far-end position 700a.

The position of the effective range 700 closest to the light emitter 202 in the x-axis direction is represented by a near-end position 700b. In a case where a plurality of such positions exist, one of these positions is set as the near-end position 700b. The portion of the incident light 211 that reaches the near-end position 700b is represented by near incident light 211b. The near incident light 211b means that the optical path from the light emitter 202 to the reflective medium 207 is the shortest. The incident angle of the incident light 211 to the reflective medium 207 corresponds to a minimum value 703b at the near-end position 700b. Of the light emitted from the light emitter 202, light not included between the far incident light 211a and the near incident light 211b attenuates as it is reflected multiple times at the light shield 704.

As illustrated in FIG. 7A, the portion of the photodetector 204 that the reflected light 212 reaches is represented by reachable range 701. The reachable range 701 is the portion of the photodetector 204 that the light emitted by the light emitter 202 and reflected at the reflective medium 207 reaches.

The reflected light of the far incident light 211a is represented by lower end reflected light 212a. The lower end reflected light 212a is, of the reflected light 212, light at the lowest position in the z-axis direction (in other words, the portion close to the diaphragm 206). The position in the reachable range 701 that the lower end reflected light 212a reaches is represented by a lower end position 701a. In the configuration of FIG. 7A, the lower end position 701a can also be referred to as, of the reachable range 701, the closest position to the diaphragm 206 in the normal direction (in other words, the z-axis direction) of the diaphragm 206 when in a non-pressed state. Also, in the configuration of FIG. 7A, the lower end position 701a can be referred to as, of the reachable range 701, the position reached by light (in other words, the lower end reflected light 212a) with the largest angle of reflection at the reflective medium 207. The maximum value of the angle of reflection is equal to the maximum value 703a of the incident angle. Also, in the configuration of FIG. 7A, the lower end position 701a can also be referred to as the farthest position from the light emitter 202 in a plan view of the diaphragm 206 when in a non-pressed state.

The reflected light of the near incident light 211b is represented by upper end reflected light 212b. The upper end reflected light 212b is, of the reflected light 212, light at the highest position in the z-axis direction (in other words, the portion far from the diaphragm 206). The position in the reachable range 701 that the upper end reflected light 212b reaches is represented by an upper end position 701b. In the configuration of FIG. 7A, the upper end position 701b can also be referred to as, of the reachable range 701, the farthest position from the diaphragm 206 in the normal direction (in other words, the z-axis direction) of the diaphragm 206 when in a non-pressed state. Also, in the configuration of FIG. 7A, the upper end position 701b can be referred to as, of the reachable range 701, the position reached by light (in other words, the upper end reflected light 212b) with the smallest angle of reflection at the reflective medium 207. The minimum value of the angle of reflection is equal to the minimum value 703b of the incident angle. Also, in the configuration of FIG. 7A, the upper end position 701b can also be referred to as the closest position to the light emitter 202 in a plan view of the diaphragm 206 when in a non-pressed state.

As illustrated in FIG. 7B, in a state where the diaphragm 206 is pressed by the body surface 300, each position of the effective range 700, the far-end position 700a, the near-end position 700b, the reachable range 701, the lower end position 701a, and the upper end position 701b changes.

As illustrated in FIGS. 7A and 7B, the upper end position 701b is defined by the portion of the light shield 705 above the reflected light 212. Thus, the portion of the light shield 704 below the incident light 211 may not block the light emitted from the light emitter 202. For example, the portion of the light shield 704 below the incident light 211 may not be provided. Also, the lower end position 701a is defined by the portion of the light shield 704 above the incident light 211. Thus, the portion of the light shield 705 below the reflected light 212 may not block the light reflected at the reflective medium 207. For example, the portion of the light shield 705 below the reflected light 212 may not be provided.

Change in Reachable Range of Reflected Light in Electronic Stethoscope

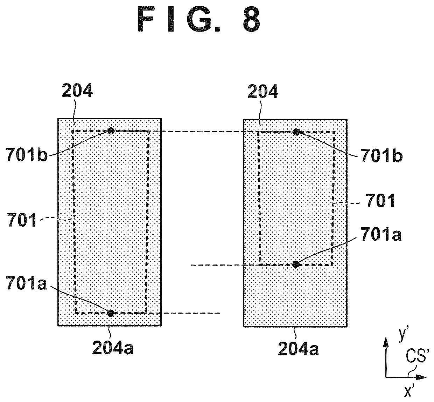

Change in the reachable range 701, which is the portion where the reflected light 212 reaches the photodetector 204 will be described with reference to FIG. 8. FIG. 8 illustrates a plan view of a light-receiving surface of the photodetector 204. On the left side of FIG. 8, a position of the reachable range 701 when the diaphragm 206 is in a non-pressed state is illustrated. On the right side of FIG. 8, a position of the reachable range 701 when the diaphragm 206 is in a state of being pressed by the body surface 300 is illustrated.

A coordinate system CS' is applied to FIG. 8 for describing the direction. The coordinate system CS' is a two-dimensional Cartesian coordinate system with an x'-axis and a y'-axis orthogonal to one another. The x'-axis is aligned with the x-axis of the coordinate system CS. The y'-axis is parallel with the yz plane of the coordinate system CS. Hereinafter, the y'-axis positive direction may be expressed as up/upper and the y'-axis negative direction may be expressed as down/lower.

The surface of the photodetector 204 that faces an internal space 213 corresponds to the light-receiving surface. An area sensor is disposed in the light-receiving surface. Using the area sensor, the photodetector 204 detects the amount of light emitted to the light-receiving surface. The light-receiving surface may have a rectangular shape. Of the four sides of the light-receiving surface, the side that is parallel with the diaphragm 206 and closer to the diaphragm 206 is represented by side 204a.

As illustrated in FIG. 8, the range (in other words, the reachable range 701) of the light reaching the photodetector 204 changes in the y'-axis direction in conjunction with movement of the reflective medium 207. Specifically, as the displacement amount of the diaphragm 206 from a flat state increases, the distance between the lower end position 701a and the upper end position 701b decreases, and the area of the reachable range 701 decreases. Thus, as the displacement amount of the diaphragm 206 from a flat state increases, the received light level by the photodetector 204 decreases. As illustrated in FIG. 8, the movement amount of the lower end position 701a in conjunction with the movement of the reflective medium 207 is greater than the movement amount of the upper end position 701b in conjunction with the movement of the reflective medium 207.

As illustrated in FIG. 8, the change in the x'-axis direction of the reachable range 701 is greater than the change in the y'-axis direction of the reachable range 701. Thus, to increase the dynamic range of the photodetector 204, it is preferable that the width of the photodetector 204 in the x'-axis direction is greater than the width of the photodetector 204 in the y'-axis direction. Specifically, the width of the photodetector 204 in the x'-axis direction is preferably three times or more the width of the photodetector 204 in the y'-axis direction.

Modification Example of Electronic Stethoscope

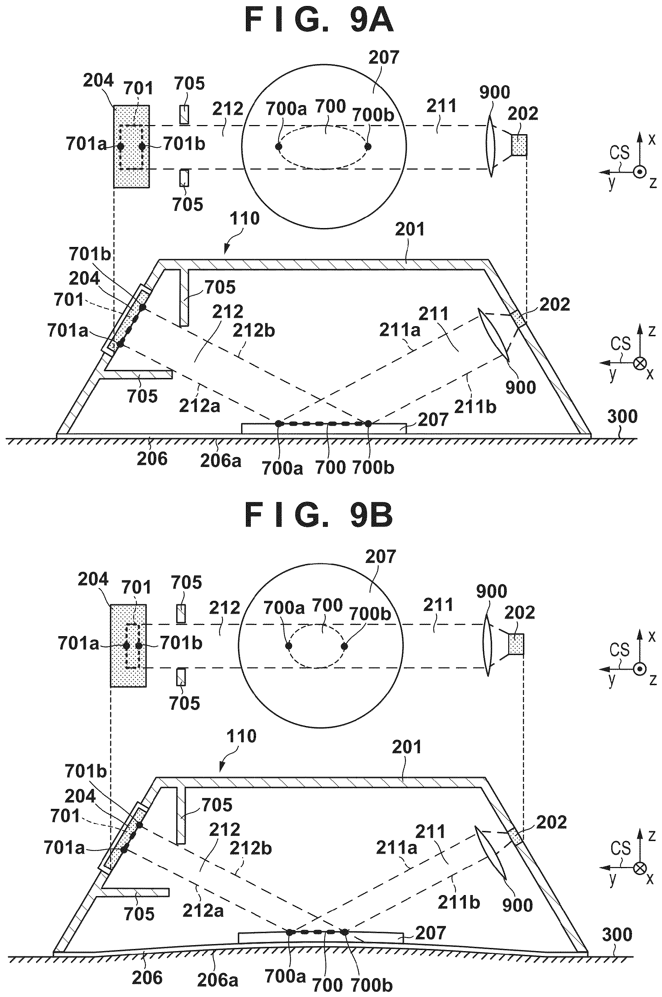

A modification example of the chest piece 110 will now be described with reference to FIGS. 9A and 9B. The differences between FIGS. 9A and 9B and FIGS. 7A and 7B will be described. Thus for points that are similar to those described using FIGS. 7A and 7B, redundant descriptions will be omitted.

In the example of FIGS. 9A and 9B, the chest piece 110 further includes a lens 900 between the light emitter 202 and the reflective medium 207. The lens 900 converts the diffused light emitted by the light emitter 202 into parallel light. Since the parallel light is emitted to the reflective medium 207, the chest piece 110 can be without a diaphragm on the incident light 211 side.

Change of the reachable range 701 is as described using FIGS. 7A and 7B. Accordingly, the range (in other words, the reachable range 701) of the light reaching the photodetector 204 changes in the y'-axis direction in conjunction with movement of the reflective medium 207.

In the embodiment described above, the surface normal of the light-receiving surface of the photodetector 204 is inclined with respect to the z-axis direction (in other words, the normal direction of the diaphragm 206). Alternatively, the surface normal of the light-receiving surface of the photodetector 204 may be aligned with the z-axis direction. In other words, the light-receiving surface is parallel with the diaphragm 206.

In the embodiment described above, the chest piece 110 includes only one light emitter 202. Alternatively, the chest piece 110 may include a plurality of the light emitters 202. The light emitted by each of the plurality of light emitters 202 may be reflected at the reflective medium 207 and reach the photodetector 204. The photodetector 204 may detect the displacement of the diaphragm 206 on the basis of the total amount of light from this light. The chest piece 110 may include a plurality of the photodetectors 204. The plurality of photodetectors 204 may each receive light emitted from each light emitter 202 and reflected at the reflective medium 207. The displacement of the diaphragm 206 may be detected on the basis of the total received light level at the plurality of photodetectors 204.

Detailed Shape of Electronic Stethoscope

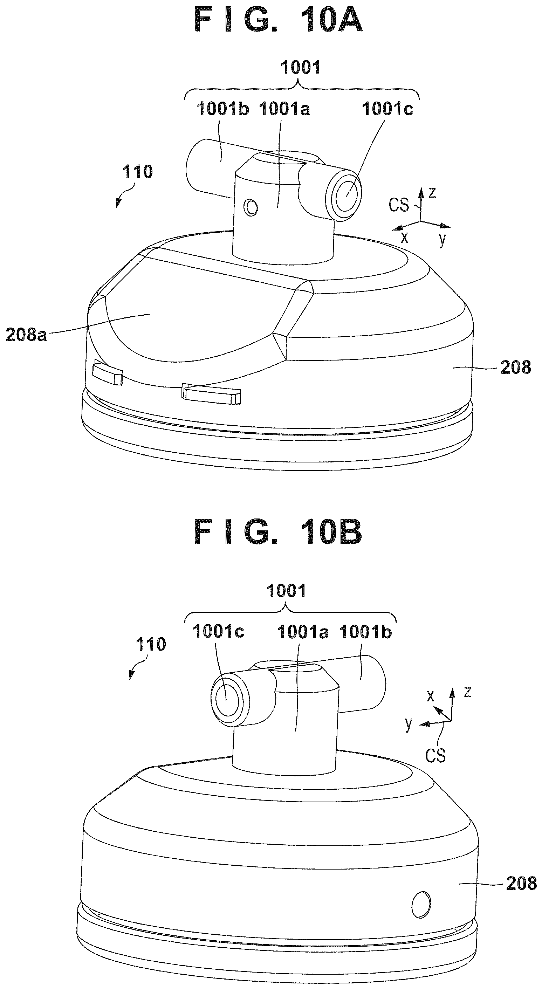

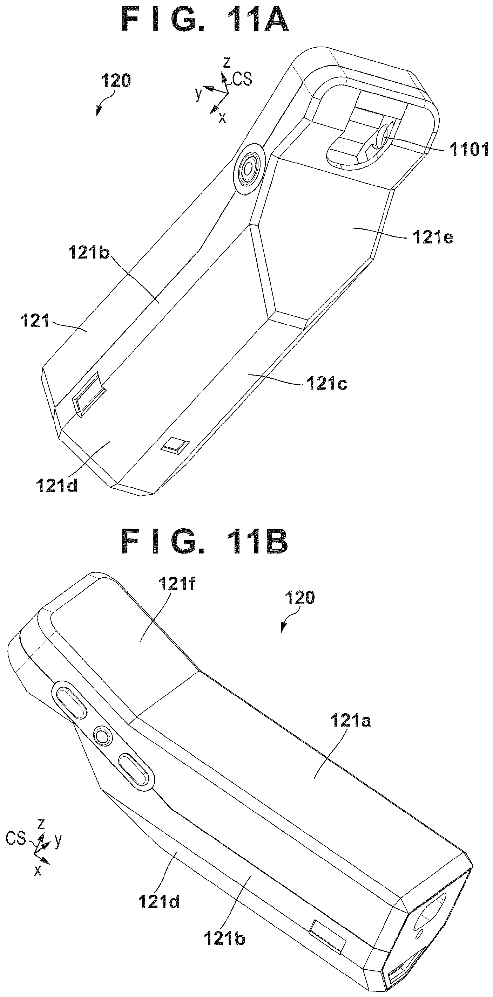

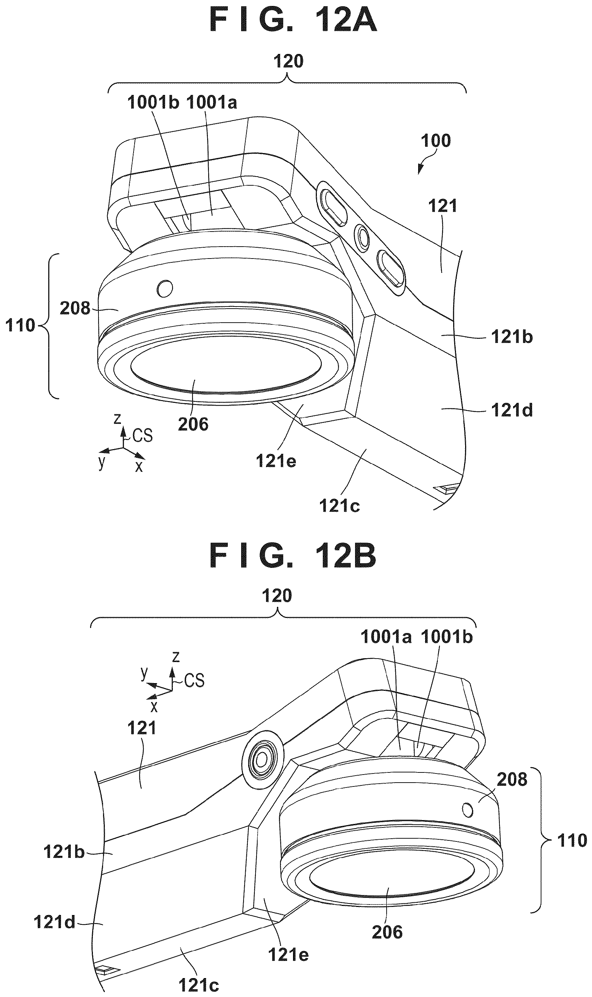

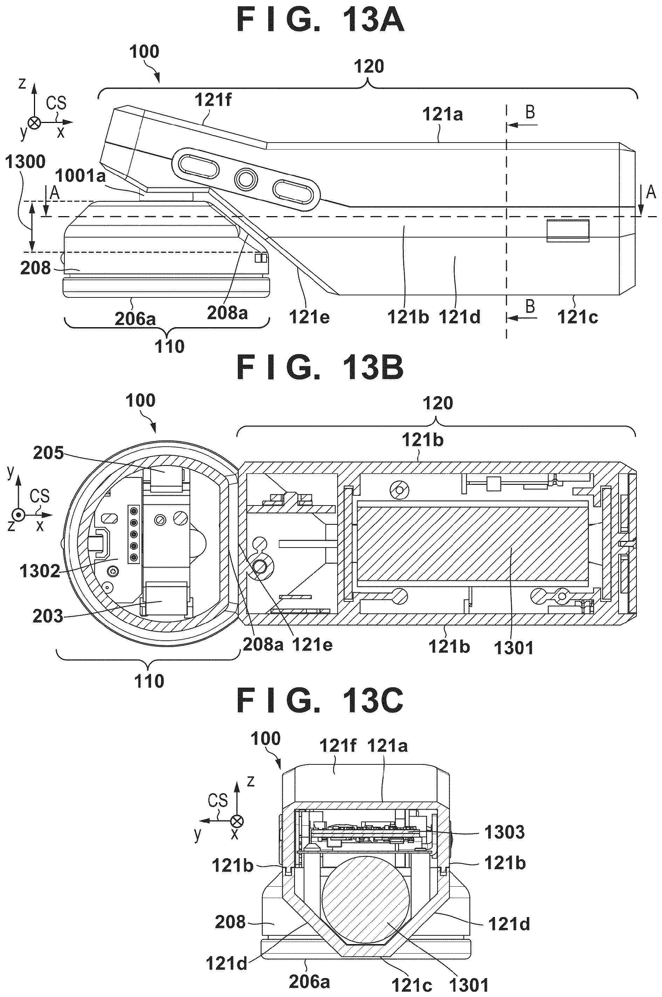

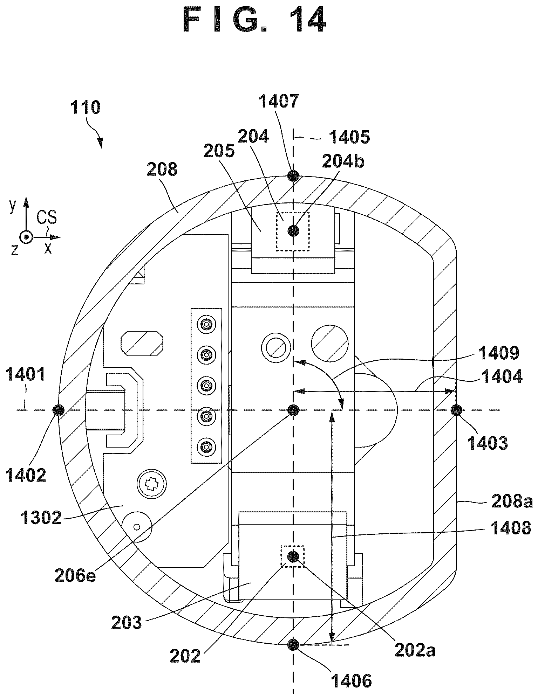

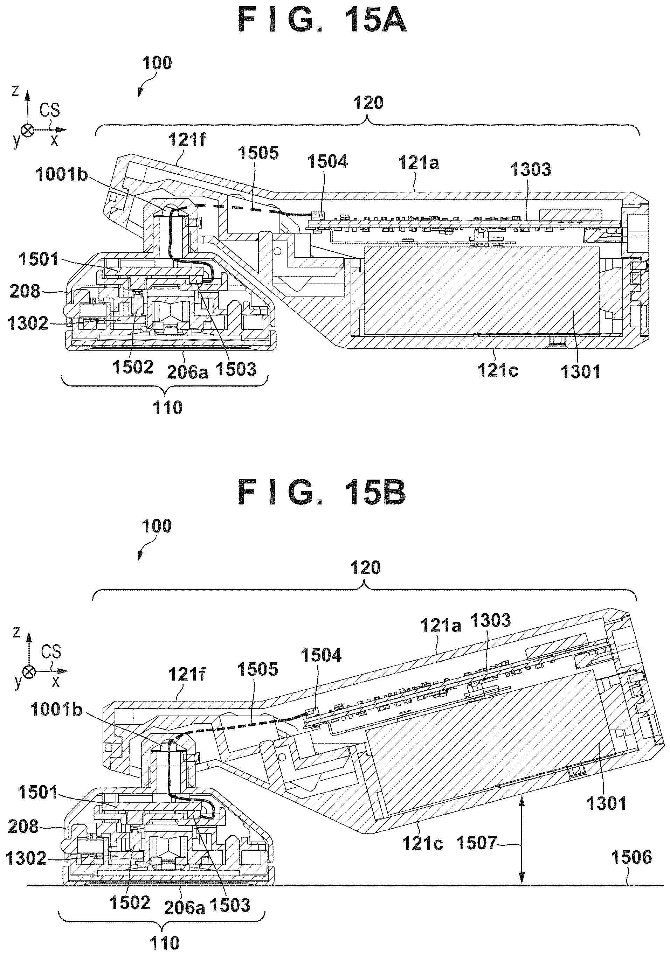

The shape of the electronic stethoscope 100 will be described in detail with reference to FIGS. 10A to 15B. FIGS. 10A and 10B are perspective views of only the chest piece 110 of the electronic stethoscope 100 as seen from different angles. FIGS. 11A and 11B are perspective views of only the grip 120 of the electronic stethoscope 100 as seen from different angles. FIGS. 12A and 12B are perspective views of the area near the chest piece 110 of the electronic stethoscope 100 as seen from different angles. FIG. 13A is a side view of the electronic stethoscope 100. FIG. 13B is a cross-sectional view of the electronic stethoscope 100 taken along line A-A of FIG. 13A. Line A-A represents a virtual cross section taken along a plane parallel with an outer surface 206a of the diaphragm 206 that at least passes through the photodetector 204. FIG. 13C is a cross-sectional view of the electronic stethoscope 100 taken along line B-B of FIG. 13A. FIG. 14 is a diagram focusing on the chest piece 110 of FIG. 13B. In FIG. 14, for the sake of visual clarity, the outer side of the cross section of the casing 208 is omitted. FIGS. 15A and 15B are cross-sectional views of the electronic stethoscope 100 taken along a plane parallel with the xz plane in cases where the chest piece 110 is at different positions with respect to the grip 120. FIG. 15A illustrates a case where the chest piece 110 is at one end of the pivotable range of the grip 120. FIG. 15B illustrates a case where the chest piece 110 is at the other end of the pivotable range of the grip 120. In other words, the chest piece 110 is pivotable (specifically, turn) between the position illustrated in FIG. 15A and the position illustrated in FIG. 15B with respect to the grip 120.

Parallel as used in the following description may be substituted with substantially parallel. "A straight line and a plane are substantially parallel" may mean that the angle formed by the straight line and the plane ranges from 0 degrees to 10 degrees. Also, "two straight lines are substantially parallel" may mean that the angle formed by the two straight lines ranges from 0 degrees to 10 degrees. Also, orthogonal as used in the following description may be substituted with substantially orthogonal. "A straight line and a plane are substantially orthogonal" may mean that the angle formed by the straight line and the plane ranges from 80 degrees to 90 degrees. Also, "two straight lines are substantially orthogonal" may mean that the angle formed by the two straight lines ranges from 80 degrees to 90 degrees. Here, "an angle formed" indicates, of the angles formed by the two, the angle included in a range from 0 degrees to 90 degrees.

As described with reference to FIGS. 1A and 1B, the grip 120 includes the casing 121 that houses components such as a battery 1301 and a main circuit board 1303. The casing 121 may be made of resin, for example. The casing 121 may have rigidity. For example, the casing 121 may maintain its shape when the user uses the electronic stethoscope 100 (for example, when the user grips the grip 120). The grip 120 has a rod-like shape, and the chest piece 110 is joined to one end of the grip 120. The direction in which the grip 120 extends is represented by the long side direction of the grip 120. In FIGS. 10A to 13C, the x-axis direction is the long side direction. For example, the long side direction of the grip 120 is the direction in which the longest side of the smallest cuboid housed inside the casing 121 of the grip 120 extends. The casing 121 may have a size that allows it to be gripped by the user. For example, the length of the casing 121 (for example, the size of the grip 120 in the long side direction) may be in a range from 50 mm to 150 mm, for example. The length around the casing 121 centered on the long side direction may be in a range from 50 mm to 200 mm, for example.

Components such as the battery 1301 and the main circuit board 1303 are housed in the casing 121 of the grip 120. The main circuit board 1303 includes a circuit element (for example, an integrated circuit, an electrode pad, a conductive pattern, or the like) for controlling the entire operations of the electronic stethoscope 100. Specifically, the main circuit board 1303 controls the operations of the chest piece 110, the operations of the operation portions 122a and 122b, and the indicators. The main circuit board 1303 may have a plate-like shape and include a mounting surface parallel with the xy plane. As described above, the long side direction of the main circuit board 1303 (for example, the direction in which the long side of the mounting surface extends) may be considered to be the long side direction of the grip 120.

The battery 1301 stores the power to be used in the electronic stethoscope 100. The battery 1301 may have a plate-like or column-like shape. The long side direction of the battery 1301 may be considered to be the long side direction of the grip 120. The battery 1301 is disposed on the lower side of the main circuit board 1303.

The chest piece 110 includes, in addition to the component elements described above, an aggregation substrate 1302 that aggregates the electrical signals of the light emitter 202 and the photodetector 204 and a relay board 1501 for relaying signals and power transferred between the chest piece 110 and the grip 120. To reduce the height of the chest piece 110, the aggregation substrate 1302 is disposed at a position off the optical path of the light emitted from the light emitter 202 in a plan view of the outer surface 206a of the diaphragm 206. On the aggregation substrate 1302, a contact 1502 for connecting to the relay board 1501 is disposed. The contact 1502 may be a retractable pin or a connector.

The chest piece 110 is joined to the grip 120 in a manner allowing the chest piece 110 to pivot with respect to the grip 120. Detailed configuration examples for enabling such a join will be described below. With a configuration different from that described below, the chest piece 110 may be able to pivot with respect to the grip 120. By the chest piece 110 being able to pivot with respect to the grip 120, the outer surface 206a of the diaphragm 206 tends to make better contact with the body surface 300 when the electronic stethoscope 100 is used. This allows the electronic stethoscope 100 to accurately detect displacement of the body surface 300.

The chest piece 110 includes a joining member 1001 attached to roughly the center of the upper surface of the casing 208. The joining member 1001 is a member separate from the casing 208. For example, the joining member 1001 may be joined to a protrusion portion formed in roughly the center of the upper surface of the casing 208. The join may be a mechanical join performed using a screw or a similar fastener or may be a chemical join performed using an adhesive or the like. The joining member 1001 may be made of the same material (for example, metal) as the casing 208 or may be made of a different material (for example, resin). Alternatively, the joining member 1001 may be a portion of the casing 208, or the joining member 1001 and the casing 208 may be integrally formed.

The joining member 1001 includes a central portion 1001a and a rotation shaft 1001b. The rotation shaft 1001b extends outward from the central portion 1001a. The rotation shaft 1001b includes two end portions located on opposite sides, and the central portion 1001a is located between these two end portions. The rotation shaft 1001b may extend parallel with the outer surface 206a of the diaphragm 206. For example, the rotation shaft 1001b extends in the y-axis direction. In a plan view of the outer surface 206a of the diaphragm 206, the axial direction of the rotation shaft 1001b is orthogonal to the long side direction of the grip 120.

The rotation shaft 1001b includes a surface with a cylindrical shape. Specifically, the cross section taken along the xz plane of the rotation shaft 1001b is circular. A hole 1001c is formed inside the shaft on one side of the rotation shaft 1001b. The hole 1001c is connected to the inside of the chest piece 110. The hole 1001c forms a path for routing a wire harness or the like connecting the circuit board inside the chest piece 110 to the circuit board inside the grip 120.

The casing 121 includes two opposing receiving portions 1101. One of the receiving portions 1101 engages with one end portion of the rotation shaft 1001b, and the other receiving portion 1101 engages with the other end portion of the rotation shaft 1001b. In this manner, the chest piece 110 is joined to the grip 120 in a manner allowing the chest piece 110 to pivot with respect to the grip 120. Specifically, with this configuration, the chest piece 110 is pivotable (in other words, can turn) with respect to the grip 120 with the rotation shaft 1001b as the center along a plane (xz plane) orthogonal to the direction that the rotation shaft 1001b extends. In the present specification, "the chest piece 110 is pivotable with respect to the grip 120" may mean that the angle of the outer surface 206a of the diaphragm 206 with respect to the long side direction of the grip 120 can change. The chest piece 110 may be pivotable with respect to the grip 120 in another manner. For example, the chest piece 110 may be pivotable with respect to the grip 120 without using a specific rotation shaft and may be pivotable by sliding on a rail including a curved portion, for example.

The receiving portions 1101 have a structure that supports the rotation shaft 1001b in a manner allowing it to turn and are recess portions or holes into which the rotation shaft 1001b is inserted. The two receiving portions 1101 are provided on opposite sides of a space, and the central portion 1001a of the joining member 1001 is disposed in this opening. The chest piece 110 is joined to the grip 120 in a manner such that the rotation shaft 1001b overlaps the diaphragm 206 in a plan view of the outer surface 206a of the diaphragm 206. This makes it easier for the user to bring the outer surface 206a of the diaphragm 206 into close contact with the body surface 300 while naturally gripping the grip 120.

The electronic stethoscope 100 includes a wire harness 1505 connecting the relay board 1501 of the chest piece 110 and the main circuit board 1303 of the grip 120. Specifically, the wire harness 1505 connects a connector 1504 mounted on the main circuit board 1303 of the grip 120 and a connector 1503 mounted on the relay board 1501 of the chest piece 110.

As described above, the hole 1001c for routing the wire harness 1505 is formed in the joining member 1001 of the chest piece 110. Also, a hole for routing the bundled wire 1505 is also formed in the receiving portion 1101 of the grip 120 (specifically, the casing 121). Since the wire harness 1505 routed through these holes is concealed by the grip 120, the possibility of the wire harness 1505 being damaged by effects from outside the electronic stethoscope 100 is reduced. Since the hole 1001c is formed on the axis line of the rotation shaft 1001b, the length of the wire harness 1505 does not greatly change depending on the position of the chest piece 110 with respect to the grip 120. Accordingly, forces applied to the wire harness 1505 and the connectors 1503 and 1504 by the chest piece 110 pivoting can be reduced.

The outer surface of the casing 208 of the chest piece 110 includes an opposing surface 208a. The opposing surface 208a is the surface that faces the grip 120 in a case where the chest piece 110 is at least at any position within the pivotable range with respect to the grip 120. For example, the opposing surface 208a faces the grip 120 in a case where the opposing surface 208a is at a position closest to the grip 120. A specific surface facing a specific object means that an outward-orientated normal vector at least at any position on the surface hits the object. In the illustrated example, the opposing surface 208a is a flat surface. Alternatively, at least a part of the opposing surface 208a may be curved.

The outer surface of the casing 121 of the grip 120 includes an upper surface 121a, a pair of side surfaces 121b, a lower surface 121c, a pair of inclined surfaces 121d, an opposing surface 121e, and a pressing surface 121f. The upper surface 121a, the side surfaces 121b, and the lower surface 121c are the surfaces located on the top, side, and bottom of the grip 120. As illustrated in FIG. 13C, the side surfaces 121b are orthogonal to the upper surface 121a, and the lower surface 121c is parallel with the upper surface 121a.

The inclined surfaces 121d are surfaces connecting the side surfaces 121b and the lower surface 121c and are inclined with respect to both of these surfaces. The portion of the grip 120 defined by the upper surface 121a, the side surfaces 121b, the lower surface 121c, and the inclined surfaces 121d is gripped by the user using the electronic stethoscope 100. By reducing the space at or near the battery 1301 by bringing the inclined surfaces 121d closer to the battery 1301, the electronic stethoscope 100 can be made both smaller and easier to grip.

The opposing surface 121e is the surface that faces the chest piece 110 in a case where the chest piece 110 is at least at any position within the pivotable range with respect to the grip 120. For example, the opposing surface 121e faces the chest piece 110 in a case where the opposing surface 208a of the chest piece 110 is at a position closest to the grip 120.

The pressing surface 121f is the surface the user of the electronic stethoscope 100 applies a force to for pressing the chest piece 110 against the body surface 300. For example, the user grips the grip 120 of the electronic stethoscope 100 with one hand and presses down on the pressing surface 121f with the other hand (for example, the index finger or the like) to bring the chest piece 110 into close contact with the body surface 300.

In the illustrated example, the upper surface 121a, the side surfaces 121b, the lower surface 121c, the inclined surfaces 121d, the opposing surface 121e, and the pressing surface 121f are all flat surfaces. Alternatively, at least a part of these surfaces may be curved.

Relationship between Optical Path and Long Side Direction

The relationship between the optical path direction of a vibration detection unit and the long side direction of the grip 120 will be described in detail with further reference to FIG. 14. As described above, the light emitter 202, the photodetector 204, and the reflective medium 207 form a vibration detection unit that detects vibration of the diaphragm 206. In a plan view of the outer surface 206a of the diaphragm 206, the direction passing through the center of a center 202a of the light emitter 202 and a center 204b of the photodetector 204 is represented by the optical path direction of the vibration detection unit. In a plan view of the outer surface 206a of the diaphragm 206, the size of the vibration detection unit in a direction intersecting the optical path direction may be smaller than the size of the vibration detection unit in the optical path direction. Thus, because the chest piece 110 is joined to the grip 120 with the optical path direction of the vibration detection unit intersecting the long side direction of the grip 120, the chest piece 110 can be brought close to the grip 120 in the long side direction of the grip 120. As a result, the size of the electronic stethoscope 100 in the long side direction (in other words, the entire length) can be reduced. In the example of FIG. 14, in a plan view of the outer surface 206a of the diaphragm 206, the optical path direction of the vibration detection unit is orthogonal to the long side direction of the grip 120.

The outer surface of the chest piece 110 illustrated in FIG. 14 has a shape that allows the chest piece 110 to be brought close to the grip 120 in the long side direction of the grip 120. The shape of the outer surface of the chest piece 110 will now be described in detail.

In the cross section of FIG. 14, the straight line extending through the center 206e of the diaphragm 206 in the long side direction (x-axis direction) of the grip 120 is represented by a straight line 1401. The straight line 1401 intersects the outer surface of the chest piece 110 at an intersection point 1402 and an intersection point 1403 (first intersection point). The intersection point 1402 is located on the opposite side of the center 206e of the diaphragm 206 from the grip 120. Also, the intersection point 1402 is located on the opposing surface 208a. The intersection point 1403 is located between the grip 120 and the center 206e of the diaphragm 206. In the cross section of FIG. 14, the distance between the center 206e of the diaphragm 206 and the intersection point 1403 is represented by a distance 1404.

In the cross section of FIG. 14, the straight line extending through the center 206e of the diaphragm 206 in the optical path direction of the vibration detection unit is represented by a straight line 1405. The straight line 1405 intersects the outer surface of the chest piece 110 at an intersection point 1406 and an intersection point 1407 (second intersection point). In the cross section of FIG. 14, the distance between the center 206e of the diaphragm 206 and the intersection point 1406 is represented by a distance 1408. In the example of FIG. 14, the distance between the center 206e of the diaphragm 206 and the intersection point 1407 is equal to the distance 1408. An angle 1409 formed by the straight line 1401 and the straight line 1405 is equal to the angle formed by the optical path direction of the vibration detection unit and the long side direction of the grip 120. In the example of FIG. 14, the angle 1409 is 90 degrees.

The distance 1404 is shorter than the distance 1408. Thus, compared to a case when the outer surface of the chest piece 110 has rotational symmetry, the chest piece 110 can be brought closer to the grip 120 in the long side direction of the grip 120. The distance 1404 being shorter than the distance 1408 is satisfied in a cross section taken along any plane parallel with the outer surface 206a of the diaphragm 206 included in a range 1300 of FIG. 13A.

In a case where the chest piece 110 is at any position (for example, the position closest to the grip 120) in a pivotable range with respect to the grip 120, the opposing surface 208a of the chest piece 110 extends along the outer surface (specifically, the opposing surface 121e) of the grip 120. Thus, the chest piece 110 can further be brought closer to the grip 120 in the long side direction of the grip 120. Specifically, the opposing surface 208a of the chest piece 110 and the opposing surface 121e of the grip 120 are both flat surfaces, and in a case where the chest piece 110 is at any position (for example, the position closest to the grip 120) in a pivotable range with respect to the grip 120, these surfaces are parallel with one another. Alternatively, the opposing surface 208a of the chest piece 110 may be a convex curved surface and the opposing surface 121e of the grip 120 may be a concave curved surface or vice versa.

In the cross section of FIG. 14, the distance between the center 206e of the diaphragm 206 and the intersection point 1402 may be longer than the distance 1404. In this manner, space can be ensured for housing components such as the aggregation substrate 1302 inside the chest piece 110.

Details of Arrangement of Pressing Surface

The arrangement of the pressing surface 121f of the grip 120 will now be described in detail with further reference to FIGS. 15A and 15B.

Hereinafter, the position illustrated in FIG. 15A is referred to as a home position of the chest piece 110, and the position illustrated in FIG. 15B is referred to as an auscultation position of the chest piece 110. The home position is a position where the chest piece 110 stops in a case where the chest piece 110 is turned anticlockwise about the rotation shaft 1001b as illustrated in FIGS. 15A and 15B. The auscultation position is a position where the chest piece 110 stops in a case where the chest piece 110 is turned clockwise about the rotation shaft 1001b as illustrated in FIGS. 15A and 15B. For example, the chest piece 110 can be turned 15 degrees or more with respect to the grip 120. For example, the upper limit of the range in which the chest piece 110 can turn with respect to the grip 120 may be 80 degrees, 90 degrees, or 100 degrees.

As illustrated in FIG. 15A, in a case where the chest piece 110 is at the home position, an angle formed by the long side direction of the grip 120 and the outer surface 206a of the diaphragm 206 is approximately 0 degrees. Accordingly, the volume required to house the electronic stethoscope 100 can be reduced.

As illustrated in FIG. 15B, in a case where the chest piece 110 is at the auscultation position, the pressing surface 121f is parallel with the outer surface 206a of the diaphragm 206. If the user presses the pressing surface 121f in this state, the pressing force is easier transferred to the diaphragm 206, allowing the diaphragm 206 to further come into close contact with the body surface 300.

Also, in a plan view of the outer surface 206a of the diaphragm 206, the pressing surface 121f overlaps the center 206e of the diaphragm 206. Furthermore, in a plan view of the outer surface 206a of the diaphragm 206, the pressing surface 121f overlaps the rotation shaft 1001b of the chest piece 110. Accordingly, a force being applied to turn the chest piece 110 with respect to the grip 120 when the user presses down the pressing surface 121f is reduced. Also, the diaphragm 206 is a flat surface and has rigidity. This makes it easy for the user to apply a force to the pressing surface 121f.

In a case where the chest piece 110 is at the auscultation position, an angle formed by the long side direction of the grip 120 and the outer surface 206a of the diaphragm 206 may be 15 degrees or greater. Specifically, in a case where the chest piece 110 is at the auscultation position, an interval 1507 of 20 mm or greater is formed between the portion of the grip 120 gripped by the user and a flat surface 1506 that passes through the outer surface 206a of the diaphragm 206. The interval 1507 may be an interval between the center of the lower surface 121c of the grip 120 and the flat surface 1506 or may be an interval between a position of the lower surface 121c of the grip 120 closest to the flat surface 1506 and the flat surface 1506. In a case where the chest piece 110 is at the auscultation position, since the user can put a finger in the interval 1507, the user can firmly grip the grip 120.

Modification Example of Electronic Stethoscope

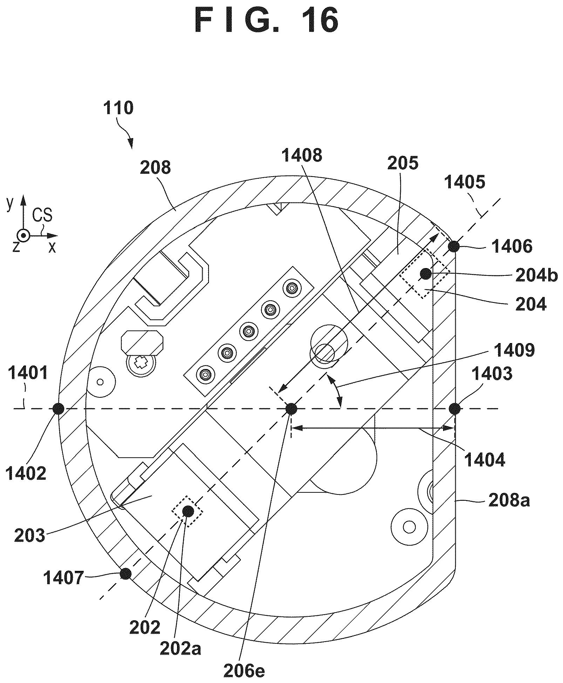

A modification example of the electronic stethoscope 100 will now be described with reference to FIG. 16. FIG. 16 illustrates a cross-sectional view of the chest piece 110 at a position corresponding to FIG. 14. In FIG. 16, the angle formed by the optical path direction of the vibration detection unit and the long side direction of the grip 120 is different from that of the example of FIG. 14. As described above, in the example of FIG. 14, the angle 1409 formed by the optical path direction of the vibration detection unit and the long side direction of the grip 120 is 90 degrees. In the example of FIG. 16, the angle 1409 is 45 degrees.

The angle 1409 is not limited to the examples described above. For example, the angle 1409 may be an angle greater than 0 degrees such as 30 degrees or more. Typically, to allow the chest piece 110 to be brought closer to the grip 120, the angle 1409 is closer to 90 degrees. The distance between the center 206e of the diaphragm 206 and the opposing surface 208a is also dependent on the size of the components housed in the chest piece 110. Thus, the angle 1409 is determined also on the basis of the size of the components housed in the chest piece 110.

Various Modification Examples

In the electronic stethoscope 100 described above, the light emitter 202, the photodetector 204, and the reflective medium 207 form a vibration detection unit that detects vibration of the diaphragm 206. Alternatively, the vibration detection unit may be configured using an element (for example, a microphone or a piezoelectric element) that detects vibrations in the air caused by the vibration of the diaphragm.

In the electronic stethoscope 100 described above, separate components, the chest piece 110 and the grip 120, are joined to one another. Alternatively, the chest piece 110 and the grip 120 may be integrally formed. Even in such a case, the size of the electronic stethoscope 100 in the long side direction can be reduced by arranging the vibration detection unit so that the optical path direction of the vibration detection unit intersects the long side direction of the grip 120 in a plan view of the outer surface 206a of the diaphragm 206.

According to the embodiments described above, a detection apparatus can have a small size.

Various embodiments have been described in detail above but it will be understood that the present disclosure is not limited to these embodiments and encompasses all modifications, variants, alternatives and equivalents falling within the scope of the appended claims.

**Claims**

1.   A detection apparatus (100) configured to detect vibration of a subject (300), the detection apparatus comprising:

a diaphragm (206) configured to be displaced in response to vibration of the subject;
a light emitter (202) configured to emit light toward the diaphragm;
a photodetector (204) configured to receive light reflected by the diaphragm and generate a signal corresponding to the reflected light;
a casing (208) configured to internally house the light emitter and the photodetector; and
a grip (120) configured to rotate about a rotation shaft (1001b) relative to the casing, the grip including a portion grippable by a user, wherein

the light emitter and the photodetector are respectively arranged at one end and another end of the casing with respect to an axial direction of the rotation shaft.

2. The detection apparatus according to claim 1, wherein
the axial direction is parallel with an optical path direction that passes through a center of the light emitter and a center of the photodetector.

3. The detection apparatus according to claim 1 or 2, wherein
in a view from a direction perpendicular to an outer surface of the diaphragm, an angle formed by a long side direction of the grip and an optical path direction that passes through a center of the light emitter and a center of the photodetector is 30 degrees or greater.

4. The detection apparatus according to claim 1 or 2, wherein
in a view from a direction perpendicular to an outer surface of the diaphragm, an optical path direction that passes through a center of the light emitter and a center of the photodetector is orthogonal to a long side direction of the grip.

5. The detection apparatus according to any one of claims 1-4, wherein
the casing is joined to the grip.

6. The detection apparatus according to any one of claims 1-5, wherein

the light emitter is a light-emitting diode, and
the detection apparatus further comprises an aperture configured to regulate light emitted from the light-emitting diode.

7. The detection apparatus according to any one of claims 1-6, wherein
in a view from a direction perpendicular to an outer surface of the diaphragm, the axial direction of the rotation shaft is orthogonal to a long side direction of the grip.

8. The detection apparatus according to any one of claims 1-7, wherein

the casing is attached to one end of the grip in a long side direction, and
in a virtual cross section that passes through at least the photodetector and is parallel with an outer surface of the diaphragm,

a straight line extending in the long side direction through a center of the diaphragm intersects an outer surface of the casing at a first intersection point between another end of the grip in the long side direction and the center of the diaphragm,
a straight line extending in the axial direction through a center of the casing intersects the outer surface of the casing at a second intersection point, and
a distance between the center of the casing and the first intersection point is shorter than a distance between the center of the diaphragm and the second intersection point.

9. The detection apparatus according to any one of claims 1-8, wherein

an outer surface of the casing includes an opposing surface that faces the grip, and
the opposing surface includes a flat surface portion extending along an outer surface of the grip.

10. An electronic stethoscope comprising:

the detection apparatus according to any one of claims 1-9; and
audio output means (610) configured to output a sound signal that is based on a signal generated by the photodetector to an external audio output device.

11. A detection apparatus (100) configured to detect vibration of a subject (300), the detection apparatus comprising:

a diaphragm (206) configured to be displaced in response to vibration of the subject;
a light emitter (202) configured to emit light toward the diaphragm;

a photodetector (204) configured to receive light reflected by the diaphragm and output a signal corresponding to the reflected light;
a casing (208) configured to internally house the light emitter and the photodetector; and
a grip (120) having an elongated shape and including a portion grippable by a user, wherein
the light emitter and the photodetector are respectively arranged at one end and another end of the casing with respect to a direction orthogonal to a long side direction of the grip and parallel with an outer surface of the diaphragm.

12. The detection apparatus according to claim 11, wherein
in a view from a direction perpendicular to the outer surface of the diaphragm, an optical path direction that passes through a center of the light emitter and a center of the photodetector is orthogonal to the long side direction of the grip.

13. The detection apparatus according to claim 11 or 12, wherein

the light emitter is a light-emitting diode, and
the detection apparatus further comprises an aperture configured to regulate light emitted from the light-emitting diode.

14. The detection apparatus according to any one of claims 11-13, wherein

an outer surface of the casing includes an opposing surface that faces the grip, and
the opposing surface includes a flat surface portion extending along an outer surface of the grip.

15. An electronic stethoscope comprising:

the detection apparatus according to any one of claims 11-14; and
audio output means (610) configured to output a sound signal that is based on a signal generated by the photodetector to an external audio output device.

# FIG. 1A

# FIG. 1B

# FIG. 2

# FIG. 3A

# FIG. 3B

# FIG. 4

# FIG. 5

# FIG. 6

**100**

ELECTRONIC STETHOSCOPE

**110**

CHEST PIECE

DISPLACEMENT SIGNAL

**610**

AUDIO OUTPUT UNIT

**618**

FILTER/AMPLIFIER

SOUND SIGNAL

**611**

A/D CONVERTER

**615**

AMPLIFIER

**612**

AMPLIFIER

**616**

VOLUME ADJUSTMENT UNIT

**613**

ENCODER

**614**

WIRELESS COMMUNICATION UNIT

**617**

WIRED COMMUNICATION UNIT

**630**

COMPUTER

**620**

AUDIO OUTPUT DEVICE

# FIG. 7A

# FIG. 7B

# F I G. 8

204

204

701b

701b

701

701

701a

701a

204a

204a

y'

CS'

x'

# FIG. 9A

# FIG. 9B

# FIG. 10A

# FIG. 10B

# F I G.  11A

# F I G.  11B

# F I G.  12A

# F I G.  12B

# F I G. 13A

# F I G. 13B

# F I G. 13C

# F I G.  14

# FIG. 15A

# FIG. 15B

# F I G. 16

| | Europäisches Patentamt European Patent Office Office européen des brevets | **EUROPEAN SEARCH REPORT** | **Application Number** EP 25 21 7572 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/211838 A1 (BILAN FRANK ALBERT [US]) 27 August 2009 (2009-08-27) | 11,13-15 | INV. A61B7/04 |
| Y | * paragraphs [0075], [0124], [0126], [0127], [0131] * <br> * figures 3a, 13 * | 1,3-10 | |
| X | CN 117 653 185 A (BEIJING KANGNING RUITONG INFORMATION TECH CO LTD) 8 March 2024 (2024-03-08) | 11,12 | |
| Y | * figures 1,5 * | 1,2,5, 7-9 | |
| X | CN 113 616 233 A (BEIJING GOLDEN CHANNEL TECH CO LTD) 9 November 2021 (2021-11-09) | 11,12 | |
| Y | * figure 5 * | 1,2,5, 7-9 | |
| Y | US 2017/251997 A1 (CHUNG HAE-IN [KR] ET AL) 7 September 2017 (2017-09-07) * paragraphs [0058], [0064], [0117], [0119] * <br> * figures 10a, 10b, 11 * | 1-10 | |

TECHNICAL FIELDS SEARCHED (IPC)

A61B

The present search report has been drawn up for all claims

| Place of search | Date of completion of the search | Examiner |
|---|---|---|
| Munich | 25 March 2026 | Hemb, Björn |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 21 7572

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2026

| Patent document cited in search report | | Publication date | Patent family member(s) | | Publication date |
|---|---|---|---|---|---|
| US 2009211838 | A1 | 27-08-2009 | US | 2009211838 A1 | 27-08-2009 |
| | | | WO | 2009108617 A2 | 03-09-2009 |
| CN 117653185 | A | 08-03-2024 | NONE | | |
| CN 113616233 | A | 09-11-2021 | NONE | | |
| US 2017251997 | A1 | 07-09-2017 | CN | 106714694 A | 24-05-2017 |
| | | | EP | 3195805 A1 | 26-07-2017 |
| | | | KR | 20160031826 A | 23-03-2016 |
| | | | US | 2017251997 A1 | 07-09-2017 |
| | | | WO | 2016043385 A1 | 24-03-2016 |

EPO FORM P0459

For more details about this annex : see Official Journal of the European Patent Office, No. 12/82

**EP 4 751 654 A1**